# EUROPEAN PATENT SPECIFICATION

(11) **EP 1 161 194 B1**
(45) Date of publication and mention of the grant of the patent: **26.04.2006**
(21) Application number: 99908968.3
(22) Date of filing: 17.03.1999
(51) Int. Cl.: A61B 19/00, A61B 17/17

(54) **IMAGING AND PLANNING DEVICE FOR LIGAMENT GRAFT PLACEMENT**
GERÄT ZUR DARSTELLUNG UND PLANUNG VON KREUZBANDERSATZOPERATIONEN
DISPOSITIF D'IMAGERIE ET DE PLANIFICATION PERMETTANT LA MISE EN PLACE D'UNE GREFFE LIGAMENTAIRE

(43) Date of publication of application: 12.12.2001
(73) Proprietor: Synthes AG Chur, 7002 Chur (CH)
(72) Inventor: SATI, Marwan, CH-4600 Olten (CH); NOLTE, Lutz, Peter, CH-3626 Hünibach (CH)
(74) Representative: Lusuardi, Werther
(86) International application number: PCT/EP1999/001778
(87) International publication number: WO 2000/054687

(56) References cited:
- EP-A- 0 603 089
- WO-A-97/29679
- US-A- 5 682 886
- US-A- 5 810 008
- US-A- 5 817 022

## Description

The present invention relates to a device for realtime computerized in-situ planning and guidance of ligament graft placement as defined in the preamble of claim 1.

Anterior Cruciate Ligament (ACL) rupture is a very common sports-related injury. Reconstruction with autogenous graft using a minimal invasive endoscopic approach has become standard in ACL replacement. This has the advantage that surgery causes minimal trauma.

Unfortunately, according to the state of the art knowledge on proper ACL placement, approximately 40% of ACL ligaments are currently being misplaced. This exposes a serious problem in the quality of a procedure that, if not performed correctly, can result in premature degeneration of knee structures that eventually requires total knee replacement.

These misplacements can partially be attributed to the restricted local endoscopic view that does not give the surgeon a global overview of ligament position as seen in standard postoperative X-rays. These limits of endoscopically-identified landmarks for consistent ligament placement have lead some experienced surgeons to strongly advocate the use of fluoroscopy as a quantitative method to verify positioning intraoperatively.

Proper ligament positioning involves many factors such as:
a) Proper drill tunnel placement with respect to anatomical landmarks;
b) Avoidance of impingement;
c) Ensuring that ligament elongation does not exceed 10% (collagen fibre rupture beyond 10% elongation);
d) Proper graft tension and position to restore knee stability; and
e) Proper graft fixation in good quality bone.

A method for the determination of the femoral point of the graft attachment in case of ACL replacement is known from the EP 0 603 089 CINQUIN. This known method concerns the determination of a femoral point of graft attachment with respect to the tibial graft attachment point such that the distance between these two points remains invariant during knee flexion and extension. This known method includes the use of a reference and a pointer both provided with energy emitting markers whose position within an on-site three-dimensional coordinate system is determined by means of a three-dimensional position measurement system e.g. an optical custom position measurement system OPTOTRAK, Northern Digital, Waterloo, On. The position measurement system measures the position of the markers with respect to the on-site three-dimensional coordinate system. Therewith, the position of the tip of the pointer is determinable by means of a computer. The method comprises the steps of:
1) Attachment of a first reference at the tibia;
2) Positioning of the pointer tip at a previously determined point T₁ and measure the position of the pointer tip with respect to the first reference;
3) Positioning of the pointer tip at several points Pᵢ at the trochlea of the femur close to that position where the invariant point is expected;
4) Calculation of the distances of point T₁ and each of the points Pᵢ;
5) Displacement of the femur with respect to the tibia and calculation of the variations of the distances between T₁ and each of the points Pᵢ;
6) Selection of that point P₁ among points Pᵢ which shows the most invariant distance.

This method measures knee movement to obtain a "functional" placement of the ligament that respects certain elongation criteria.

The disadvantage of this known method is that anatomical placement criteria such as a general overview of graft position with respect to the whole anatomy of the joint is not possible.

On this point, the invention intends to provide remedial measures.

The objective of the invention is to provide a device that can support both functional and anatomical criteria for a variety of graft types, surgical philosophies and surgical techniques.

The invention solves the posed problem with a device for realtime computerized in-situ planning and guidance of ligament graft placement offering the features of claim 1.

Additional advantageous embodiments of the invention are characterized in the dependent claims.

The device according to the invention provides the advantage that the device provides a versatility that allows anterior cruciate ligament insertion for very different surgical techniques.

The device according to the invention is defined in claim 1.

The markers may be energy emitting, receiving or reflecting means depending on the position measurement device being used. For instance as energy emitting means:
- Light sources;
- Light emitting diodes (LED's);
- Infrared light emitting diodes (IRED's);
- Accoustic transmitters; or
- Coils in order to establish a magnetic field;
or as energy receiving means:
- Photodiodes;
- Microphones; or
- Hall-effect components;
may be installed.

Furthermore, the device may comprise a drilling device which is provided with at least three markers as well. The markers attached to the drilling device allow to determine the in-situ position of the drilling device, particularly of the drill tip. Such the drill tip may be positioned on the bone as previously planned at the computer. The path of the hole being bored by the drill may be controlled at the computer therewith allowing a guidance of the drilling device according to the holes previously planned at the computer. The planning of the positions of the holes may be performed with use of the medical images.

In a preferred embodiment of the device according to the invention the pointer and the endoscope are configured as a one-piece computer-integrated endoscopic instrument.

Preferably, the ultrasound device is an A-mode ultrasound device emitting and receiving an ultrasound beam along an axis such allowing a realtime signal processing.

Particular software allows the computer to display a three-dimensional representation of the connection between ligament attachment points previously determined via the pointer. Moreover, when using the ligament attachment points the computer can display a ligament during knee flexion and extension.

A method which can practiced with the device according to the invention comprises the steps
A) making at least one X-ray of the knee sections of the femur and the tibia as a medical image;
B) transfer of the medical image to a computer;
C) measurement of points on the surface of the femur respectively of the tibia by means of a pointer and within a on-site three-dimensional coordinate system;
D) measurement of points on the surface of the femur respectively of the tibia by means of an ultrasound device and within a on-site three-dimensional coordinate system; and
E) functional and anatomical determination of the ligament graft placement by means the data received under A), C) and D).

The points intraoperatively measured on the surface of the femur-respectively of the tibia may be used to establish a mathematical relationship between the patient's intraoperative position and the data of the X-rays.

The X-rays may be made preoperatively or intraoperatively and are used to help decide where to place the ligament.

Furthermore, the method may comprise the step of determination of the image magnification by the computer.

Preferably the X-rays are prepared as templates. Such allowing a direct planning of the desired ligament graft placement with respect to anatomical landmarks on the medical image displayed at the computer through manipulation of the templates.

The method may also comprise the implantation of fiducial markers at the femur respectively at the tibia.

The invention is explained in more detail with reference to the partially schematic illustration of the preferred embodiment.

Shown are:

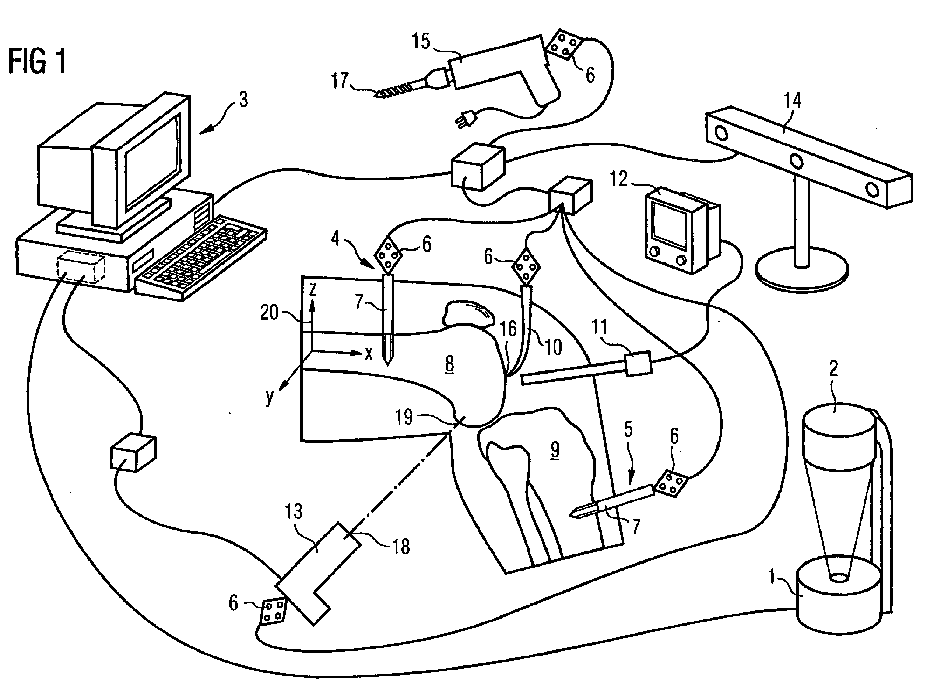

Fig. 1 the preferred embodiment of the device according to the invention.

Fig. 1 shows the device according to the invention comprising:
- An X-ray source 1 that may be used pre-operatively or intra-operatively;
- An X-ray receiver 2 for gathering medical image data;
- A computer 3 whereto the medical image data is transferred either directly in numerical format or by scanning the radiographic film into the computer 3;
- Two rigid reference bodies 4;5 with four markers 6 each and screws 7 or pins to attach the reference bodies 4;5 to the femur 8 respectively to the tibia 9;
- A pointer 10 with twelve markers 6;
- An endoscope 11 which is provided with a display 12;
- An A-mode ultrasound device 13 which is connected to the computer 3 and provided with four markers 6;
- A position measurement device 14 connected to the computer 3 in order to determine the position of the markers 6; and
- A drilling device 15 which is provided with four markers 6.

X-rays may be established pre-operatively or intra-operatively by means of the X-ray source 1 and the X-ray receiver 2. If pre-operative X-rays are taken, these may be available in numerical format or as a radiographic film. If available in numerical format, the X-ray is directly transferred to the computer 3, otherwise the radiographic film is scanned into the computer 3. If intra-operative X-rays are taken, a fluoroscope is used with an X-ray source 1 and an X-ray receiver 2. X-rays should be taken in standard orientations, for example anterior-posterior and medial-lateral, and contain a ruler with radio-opaque markings placed at the side of the knee. The radio-opaque markings on the ruler appear on the image and are digitised in the computer 3 to determine image magnification. The X-rays can be prepared as templates. Desired ligament placement with respect to anatomical landmarks, e.g. the medial and lateral condyles or the trochlea, can be planned directly on the medical image displayed at the computer 3 through manipulations of the templates.

To perform the functional considerations the position measurement of the femur 8, the tibia 9, and the pointer 10 is performed by the position measurement device 14. In the preferred embodiment of the device according to the invention the position measurement device 14 is an optoelectronic navigation system (Optotrak 3020, Northern Digital, CAN.). The markers 6 are infrared light emitting diodes (IRED's) that are detected by the position measurement device 14 therewith allowing to calculate the position of the reference bodies 4;5, the pointer tip 16, the drill tip 17 and the head 18 of the ultrasound device 13.

The pointer 10 can be used to digitise structures of the femur 8 respectively of the tibia 9 under direct visual control by means of the endoscope 11. In the preferred embodiment of the device according to the invention the pointer 10 is a computer-integrated endoscopic palpation hook such that the endocsope 11 is integrated in the pointer 10.

Certain landmarks, such as the posterior femoral and tibial condyles, are difficult to obtain by direct digitization with the pointer 10. For this reason, the device according to the invention also incorporates an A-mode ultrasound device 13 equipped with markers 6. The ultrasound device 13 and the computer 3 allow to calculate the distance between the ultrasound device head 18 and the point of intersection 19 of the ultrasound beam with the surface of a bone. Since the position of the ultrasound device head 18 and the direction of the ultrasound beam within the on-site three-dimensional coordinate system 20 are determined by the position measurement device 14 the position of the point of intersection 19 may be determined within the on-site three-dimensional coordinate system 20 by means of a coordinate transformation performed via the computer 3.

The points intraoperatively measured on the surface of the femur 8, respectively on the tibia 9 may be used to establish a relationship between the patient's intraoperative position and the data of the medical images. Such a transformation of coordinates on the medical image may be computed into coordinates within the on-site three-dimensional coordinate system 20 therewith allowing functional and anatomical considerations of the graft placement.

## Claims

1. Device for realtime computerized in-situ planning and guidance of ligament graft placement comprising
A) two rigid reference bodies (4;5) with at least three markers (6) each and screws (7) or pins to attach the reference bodies (4;5) to the femur (8) respectively to the tibia (9);
B) a pointer (10) with at least three markers (6);
C) an endoscope (11);
D) a computer (3); and
E) a position measurement device (14) connected to the computer (3) in order to determine the position of the markers (6),
**characterized in that**
the device further comprises
F) an X-ray source (1) that may be used pre-operatively or intra-operatively;
G) an X-ray receiver (2) for gathering medical image data which is transferred to the computer (3) either directly in numerical format or by scanning the radiographic film into the computer (3); and
H) an ultrasound device (13) which is connected to the computer (3) and provided with at least three markers (6), and **in that** the computer (3) is configured
I) to obtain the data indicative of the location for ligament graft placement based on positions of the pointer (10) on a femur (8), respectively a tibia (9) and based on the medical image data gathered by means of the X-ray receiver (2); and
K) to display a three dimensional representation of a ligament during knee flexion and extension by using the ligament attachment points determined with the pointer (10).

2. Device according to claim 1, **characterized in that** the device further comprises a drilling device (15) which is provided with at least three markers (6).

3. Device according to claim 1 or 2, **characterized in that** the pointer (10) and the endoscope (11) are configured as a one-piece computer-integrated endoscopic instrument.

4. Device according to one of the claims 1 through 3, **characterized in that** the ultrasound device (13) is an A-mode ultrasound device.

5. Device according to one of the claims 1 through 4, **characterized in that** the X-ray source (1) and the X-ray receiver (2) are configured as a fluoroscope.

## Patentansprüche

1. Vorrichtung zur in Echtzeit erfolgenden, computergestützten In-situ-Planung und -Führung einer Bandtransplantatplazierung, welche folgendes umfasst:
A) zwei starre Bezugskörper (4;5) mit je zumindest drei Markern (6) und mit Schrauben (7) oder Stiften, um die Bezugskörper (4;5) an dem Femur (8) bzw. an der Tibia (9) zu befestigen;
B) einen Pointer (10) mit zumindest drei Markern (6);
C) ein Endoskop (11);
D) einen Computer (3); und
E) eine mit dem Computer (3) verbundene Positionsmessungsvorrichtung (14) zur Positionsbestimmung der Marker (6),
**dadurch gekennzeichnet, dass**
die Vorrichtung weiterhin folgendes umfasst:
F) eine Röntgenquelle (1), welche präoperativ oder intraoperativ verwendet werden kann;
G) einen Röntgenempfänger (2) zur Gewinnung von medizinischen Bilddaten, welche an den Computer (3) übertragen werden, und zwar entweder direkt in numerischem Format oder durch Einscannen des Röntgenfilms in den Computer (3); und
H) eine Ultraschallvorrichtung (13), welche mit dem Computer (3) verbunden ist und mit zumindest drei Markern (6) ausgestattet ist;
und **dadurch**, dass
der Computer (3) entsprechend ausgelegt ist,
I) um die Daten zu erhalten, welche die Stelle für die Bandtransplantatplazierung anzeigen, und zwar basierend auf Positionen des Pointers (10) auf einem Femur (8) bzw. einer Tibia (9) und basierend auf den mittels des Röntgenempfängers (2) gewonnenen medizinischen Bilddaten; und
K) um durch die Verwendung der mit dem Pointer (10) bestimmten Bandbefestigungspunkte eine dreidimensionale Darstellung eines Bandes während der Kniebeugung und -streckung anzuzeigen.

2. Vorrichtung nach Anspruch 1, **dadurch gekennzeichnet, dass** die Vorrichtung weiterhin eine Bohrvorrichtung (15) umfasst, welche mit zumindest drei Markern (6) ausgestattet ist.

3. Vorrichtung nach Anspruch 1 oder 2, **dadurch gekennzeichnet, dass** der Pointer (10) und das Endoskop (11) als einteiliges, computerintegriertes endoskopisches Instrument ausgelegt sind.

4. Vorrichtung nach einem der Ansprüche 1 bis 3, **dadurch gekennzeichnet, dass** es sich bei der Ultraschallvorrichtung (13) um eine A-Mode-Ultraschallvorrichtung handelt.

5. Vorrichtung nach einem der Ansprüche 1 bis 4, **dadurch gekennzeichnet, dass** die Röntgenquelle (1) und der Röntgenempfänger (2) als Fluoroskop ausgelegt sind.

## Revendications

1. Dispositif permettant la planification et le guidage in situ assistés par ordinateur et réalisés en temps réel du placement d'une greffe ligamentaire, ledit dispositif comprenant :
A) deux corps de référence rigides (4;5) comportant respectivement au moins trois marqueurs (6) et comportant des vis (7) ou des tiges afin de fixer les corps de référence (4;5) sur le fémur (8) ou sur le tibia (9);
B) un pointeur (10) comportant au moins trois marqueurs (6);
C) un endoscope (11);
D) un ordinateur (3); et
E) un dispositif de mesure de position (14) relié audit ordinateur (3) et permettant de déterminer la position des marqueurs (6),
**caractérisé en ce que**
le dispositif comprend en outre :
F) une source de rayons X (1) qui peut être utilisée avant ou pendant l'opération;
G) un récepteur de rayons X (2) permettant d'obtenir des données d'images médicales, lesquelles sont transmises à l'ordinateur (3), soit directement en format numérique, soit par scannage du film radiographique dans l'ordinateur (3); et
H) un dispositif ultrasonique (13) relié à l'ordinateur (3) et équipé d'au moins trois marqueurs (6);
et **en ce que**
l'ordinateur (3) est conçu de manière à
I) recevoir les données qui indiquent l'endroit pour le placement de la greffe ligamentaire, et ce en se basant sur les positions occupées par le pointeur (10) sur un fémur (8) ou sur un tibia (9) ainsi que sur les données d'images médicales obtenues au moyen du récepteur de rayons X (2); et
K) indiquer, en utilisant les points de fixation du ligament déterminés à l'aide du pointeur (10), une représentation tridimensionnelle d'un ligament pendant la flexion et l'extension du genou.

2. Dispositif selon la revendication 1, **caractérisé en ce que** le dispositif comprend en outre un dispositif de perçage (15) équipé d'au moins trois marqueurs (6).

3. Dispositif selon la revendication 1 ou 2, **caractérisé en ce que** le pointeur (10) et l'endoscope (11) sont conçus en une seule pièce en tant qu'instrument endoscopique intégrant un ordinateur.

4. Dispositif selon l'une des revendications 1 à 3, **caractérisé en ce que** le dispositif ultrasonique (13) est un dispositif ultrasonique mode A.

5. Dispositif selon l'une des revendications 1 à 4, **caractérisé en ce que** la source de rayons X (1) et le récepteur de rayons X (2) sont conçus en tant que fluoroscope.
